# EUROPÄISCHE PATENTSCHRIFT

(11) **EP 1 684 846 B1**
(45) Veröffentlichungstag und Bekanntmachung des Hinweises auf die Patenterteilung: **19.03.2008**
(21) Anmeldenummer: 04797585.9
(22) Anmeldetag: 04.11.2004
(51) Int. Cl.: A61M 37/00, A61N 1/30

(54) **Vorrichtung zur transdermalen Verabreichung von Wirkstoffen**
Device for the transdermal administration of active ingredients
Dispositif d'administration transdermique de substances actives

(30) Priorität: 17.11.2003 DE 10353629
(43) Veröffentlichungstag der Anmeldung: 02.08.2006
(73) Patentinhaber: LTS LOHMANN Therapie-Systeme AG, 56626 Andernach (DE)
(72) Erfinder: HORSTMANN, Michael, 56564 Neuwied (DE)
(74) Vertreter: Flaccus, Rolf-Dieter
(86) Internationale Anmeldenummer: PCT/EP2004/012458
(87) Internationale Veröffentlichungsnummer: WO 2005/049128

(56) Entgegenhaltungen:
- WO-A-20/05000382
- WO-A1-97/48440
- WO-A1-20/04033021
- GB-A- 1 315 796
- NL-A- 6 614 673
- US-B1- 6 334 856

## Beschreibung

Die vorliegende Erfindung betrifft Vorrichtungen, welche die transdermale Verabreichung von Wirkstoffen ermöglichen. Sie betrifft ferner die Verwendung solcher Vorrichtungen zur transdermalen Verabreichung von Wirk- oder Hilfsstoffen an Mensch oder Tier.

Transdermale therapeutische Systeme haben inzwischen als Darreichungsform zur Behandlung zahlreicher Erkrankungen eine weite Verbreitung gefunden. Insbesondere die Wirkstoffe Nicotin, Nitroglycerin, Scopolamin und Estradiol, aber auch viele neuere Wirkstoffe, können mittels dieser Technologie in kontrollierter und zeitlich protrahierter Form zur Wirkung gebracht werden. Besonders vorteilhaft ist dabei, dass der physiologische First-Pass-Effekt, der bei oraler Gabe stets auftritt, mit dieser Darreichungsform bei vielen Wirkstoffen weitgehend unterdrückt werden kann. Dadurch kann letztendlich ein höherer Anteil des Wirkstoffs zum Einsatzort gelangen.

Transdermale therapeutische Systeme (TTS) sind üblicherweise so aufgebaut, dass sie ein haftklebendes Wirkstoffreservoir aufweisen oder jedenfalls mindestens eine haftklebende Schicht zur Befestigung des Systems auf der Haut vorhanden ist. Die klebende Verbindung des Systems mit der Haut kommt aufgrund der selbstklebenden Eigenschaften der verwendeten Polymere oder durch geeignete Mischungen von Polymeren und Hilfsstoffen zustande.

Die haftklebende Befestigung auf der Haut, welche ausschließlich auf dem Phänomen der Oberflächenspannung beruht, ist jedoch nicht immer verlässlich. Aus diesem Grund werden des öfteren Probleme hinsichtlich der Verankerung von TTS auf der Haut beobachtet. Die unzureichende Verankerung wirkt sich vor allem auf die maximale Applikationsdauer aus; im allgemeinen ist die Verwendbarkeit solcher Systeme auf der Haut auf maximal ca. 7 Tage begrenzt.

Die Wirkstoffabgabe erfolgt bei TTS im allgemeinen durch Diffusion des Wirkstoffs durch die polymerhaltigen und weichplastischen Schichten des TTS, wobei der Wirkstoff über die Hautkontaktseite des TTS zur Hautoberfläche gelangt. Anschließend diffundieren die Wirkstoffmoleküle zunächst durch die äußerste Hautschicht (stratum corneum) und gelangen dann in die tieferen Bereiche der Epidermis, wo sie in den Blutkreislauf überführt werden.

Der soeben beschriebene diffusive Wirkstoff transport erfolgt jedoch nur bei einer sehr kleinen Anzahl von Wirkstoffen mit einer für therapeutische Zwecke ausreichenden Geschwindigkeit. Der Grund hierfür liegt vor allem in der natürlichen Barriere-Wirkung des Stratum corneum. Durch diese Barriere-Wirkung wird insbesondere die transdermale Verabreichung hochmolekularer Wirkstoffe (z. B. Peptide, Proteine, Oligo- und Polynucleotide) oder stark polarer Wirkstoffe erheblich erschwert oder unmöglich gemacht.

Es hat deshalb nicht an Versuchen gefehlt, die Barriere-Eigenschaft des Stratum corneum mittels geeigneter Methoden zu durchbrechen. Dies kann beispielsweise durch Anwendung von chemischen Permeationsverstärkern (z. B. Ethanol oder andere Alkohole), elektrischen Spannungsdifferenzen (z. B. Iontophorese) oder auch durch mechanische Veränderung des Stratum corneum bewirkt werden. Nach US 6 334 856 kann hierfür eine Vorrichtung mit einer Vielzahl von Mikro-Hohlnadeln, die in einem Feld angeordnet sind, verwendet werden. Diese Nadeln sind zwar sehr konturscharf, dringen aber nur einige hundert Mikrometer tief in die Haut ein. Auf diese Weise wird zwar die Verabreichung wirkstoffhaltiger Flüssigkeiten unter Umgehung der Barriere-Eigenschaft des Stratum corneum ermöglicht, jedoch ist es in vielen Fällen nicht möglich, die Wirkstoffe in flüssiger Form bereitzustellen. Ursache hierfür kann z. B. die mangelnde Löslichkeit des Wirkstoffs oder die mangelnde Stabilität der Wirkstofflösung sein. Außerdem kommt wegen des geringen Flüssigkeitsvolumens, das über diese Mikronadeln appliziert werden kann, nur eine sehr geringe Wirkstoffmenge (im Bereich von wenigen Mikrogramm) zur Anwendung.

Eine sehr ähnliche Vorrichtung wie in US 6 334 856 wird in US 6 083 196 beschrieben. Diese umfaßt eine Trägerfolie, auf der eine Vielzahl von Mikro-Protrusionen angeordnet sind, die zum Eindringen in die Haut geeignet sind. Die Vorrichtung wird mittels eines zusätzlich vorhandenen Befestigungsmittels (z. B. in Gestalt eines Überpflasters) auf der Haut fixiert, da die Vorrichtung als solche nicht auf der Haut haften bleibt.

WO 97/48440 A1 offenbart eine Vorrichtung zum Durchstechen des Stratum corneum einer Körperoberfläche mit mehreren, an einer Platte verbundenen Mikroklingen zur transdermalen Verabreichung von Wirkstoffen oder Probennahme von Körperanalyten.

WO 2005/000382 A2 offenbart eine Vorrichtung, mit der mittels rotierender Mikronadeln Löcher von bestimmter Tiefe und bestimmtem Durchmesser in ein vorbehandeltes Areal Haut erzeugt werden. Dabei austretende Körperflüssigkeit kann über die Mikronadeln aufgenommen werden bzw. Wirkstofflösung kann über einen Vorratsbehälter, der mit der Vorrichtung verbunden ist, in die Haut eingebracht werden.

Aufgabe der vorliegenden Erfindung war es deshalb, eine Vorrichtung zur transdermalen Verabreichung von Wirkstoffen bereitzustellen, die insbesondere für die Verabreichung höhermolekularer oder stark polarer Wirkstoffe geeignet ist, und bei welcher die beschriebenen Nachteile des Standes der Technik vermieden oder vermindert werden. Insbesondere soll dadurch die transdermale Verabreichung von Wirkstoffen ermöglicht werden, die nicht in flüssiger Form vorliegen, und die Haftung der Vorrichtung auf der Haut soll auch ohne zusätzliche selbstklebende Mittel ermöglicht werden.

Diese Aufgabe wird überraschenderweise gelöst durch eine Vorrichtung nach Anspruch 1, welche gemäß Oberbegriff die allgemeinen Merkmale eines transdermalen therapeutischen Systems aufweist, und bei der die hautseitige Kontaktfläche eine Vielzahl von stift- oder nadelförmigen Mikroprotrusionen aufweist, die zum Eindringen in die Haut geeignet sind. Diese Mikroprotrusionen sind mit Strukturen ausgestattet, welche das Herausziehen der Protrusionen aus der Haut erschweren.
Die Mikroprotrusionen der erfindungsgemäßen Vorrichtung zeichnen sich somit dadurch aus, dass zum Einstechen der Mikroprotrusionen in die Haut eine geringere Kraft erforderlich ist als zum nachfolgenden Herausziehen aus der Haut.

Durch die in die Haut eindringenden Mikroprotrusionen wird die Barriere des Stratum corneum durchbrochen und die im Reservoir enthaltenen Wirkstoffe können unter Umgehung dieser Barriere in die tieferen Bereiche der Epidermis gelangen, nachdem sie aus dem Reservoir zur Hautkontaktseite der Vorrichtung diffundiert sind. Die erfindungsgemäßen Systeme ermöglichen die Applikation von Wirkstoffen in tiefer gelegene Hautschichten, d. h. unterhalb der Barriere-Schicht des Stratum corneum.

Durch die genannten Strukturen, welche das Herausziehen der Protrusionen aus der Haut erschweren, wird eine Verankerung der Vorrichtung an der Applikationsstelle auf der Haut ermöglicht, wobei für diese Verankerung keine zusätzlichen Mittel benötigt werden. Es handelt sich um eine Fixierung, die zwar einer Verklebung ähnlich ist, die jedoch auf einer mechanischen Verankerung beruht.

Die Mikroprotrusionen sind am distalen (d. h. hautseitigen) Ende vorzugsweise zugespitzt, um das Eindringen in die Haut zu erleichtern. Bevorzugt sind sie nadelförmig gestaltet und verjüngen sich zum distalen Ende hin. Bei entsprechend geringem Querschnitt oder Durchmesser der Mikroprotrusionen ist ein Eindringen in die Haut auch möglich, wenn diese nicht zugespitzt oder verjüngt sind. Die Mikroprotrusionen können einen im wesentlichen runden, elliptischen, dreivier- oder vieleckigen, oder einen unregelmäßigen Querschnitt aufweisen. Ein sehr schmaler Querschnitt, wobei die Mikroprotrusionen annähernd die Form eines Sägemessers oder einer gezackten Klinge haben, sollte vermieden werden, weil dadurch keine ausreichende Verankerung in der Haut erreicht werden kann.
Die Dicke der Protrusionen liegt vorzugsweise im Bereich von 5 bis 100 µm, insbesondere im Bereich von 10 bis 50 µm. Bei nadelförmigen Protrusionen beträgt die Dicke an der Spitze vorzugsweise 1 bis 10 µm und an dem gegenüberliegenden Ende vorzugsweise 5 bis 100 µm.

Die geeignete Länge der Mikroprotrusionen hängt ab von der Gesamt-Dicke der Vorrichtung, insbesondere des Wirkstoffreservoirs, und von der erwünschten Eindringtiefe. Vorzugsweise haben die Mikroprotrusionen eine Länge im Bereich von 50 bis 500 µm, besonders bevorzugt im Bereich von 100 bis 300 µm. Dabei wird ferner bevorzugt, dass sie um weniger als 300 µm, insbesondere um weniger als 200 µm, aus der hautseitigen Oberfläche der Vorrichtung (z. B. der Polymermatrixschicht) herausragen.

Die genannten Strukturen, welche das Herausziehen aus der Haut erschweren und der Verankerung dienen, sind an dem äußeren Umfang der Protrusionen angeordnet. Die Verankerungsstrukturen können auch in Vielzahl (2 oder mehr) an jeweils einer Protrusion vorhanden und können über deren gesamte Länge verteilt sein. Zumindest aber ist derjenige Bereich der Protrusionen, der aus der Oberfläche der Hautkontaktseite der Vorrichtung herausragt, mit derartigen Strukturen versehen.

Die Verankerungsstrukturen können dadurch verwirklicht werden, dass die Längskontur der Mikroprotrusionen mit Hinterschneidungen versehen wird. Besonders bevorzugt wird eine Ausgestaltung dieser Strukturen in Form von Widerhaken, welche einem Herausziehen der Protrusionen aus der Haut entgegenwirken. Eine Mikroprotrusion kann jeweils einen oder vorzugsweise mehrere derartiger Widerhaken aufweisen.

Eine weitere Ausführungsform der Erfindung sieht vor, dass die Mikroprotrusionen, oder zumindest eine Teilmenge davon, schraubenförmig gestaltet und drehbar angeordnet sind. Dadurch kann durch Anwendung einer rotierenden Bewegung das Eindringen in die Haut erleichtert und eine Verankerung in der Haut bewirkt werden. Der Drehantrieb kann beispielsweise durch mikromechanische Mittel, insbesondere durch mikromechanische Aktoren, bereitgestellt werden.
Im allgemeinen haben sämtliche Mikroprotrusionen eine Vorrichtung im wesentlichen die gleiche Form; die Erfindung umfaßt aber auch solche Ausführungsformen der Vorrichtung, bei denen zwei oder mehrere Gruppen unterschiedlich gestalteter Mikroprotrusionen vorhanden sind.

Die Anzahl der Mikroprotrusionen beträgt vorzugsweise 1 bis 10³ pro mm², besonders bevorzugt 10 bis 100 pro mm².
Die Mikroprotrusionen sind mit der Rückschicht fest verbunden, oder/und sie sind in dem wirkstoffhaltigen Reservoir der Vorrichtung, das vorzugsweise als Polymer-Matrix gestaltet ist, eingebettet und befestigt. Alternativ können die Mikroprotrusionen auch auf oder in einer wirkstoffdurchlässigen Folie oder Membran angeordnet sein, die auf die hautseitige Oberfläche des Wirkstoffreservoirs aufkaschiert wird, so dass die vorzugsweise spitzen Enden der Mikroprotrusionen nach außen, d. h. zur Haut hin, gerichtet sind. Die genannte Membran oder Folie kann auch haftklebende Eigenschaften haben.

Insbesondere wenn die Mikroprotrusionen an einer halbstarren Stützfolie als Rückschicht verankert sind, ergibt sich der weitere Vorteil, dass die nach dem Widerhaken-Prinzip geformten Mikroprotrusionen bei spontanen Hautbewegungen dazu veranlasst werden, noch tiefer in die Haut einzudringen, da die Relativbewegungen der Haut über die Rückschicht oder Stützfolie auf die Mikroprotrusionen übertragen werden.

Gemäß einer weiteren Ausführungsform können die mit widerhaken-ähnlichen Strukturen ausgestatteten Mikroprotrusionen auch einen inneren Hohlraum oder Kanal aufweisen, in Form einer Hohlnadel mit einer Öffnung am distalen, hautseitigen Ende. Der Hohlraum oder Kanal steht mit einem flüssigkeitsgefüllten Reservoir in Verbindung, in welches die Hohlnadeln eintauchen oder eingebettet sind.

Alternativ können die Mikroprotrusionen auch aus einem diffusiblen Material hergestellt sein, welches die Diffusion von Wirkstoffen aus dem Reservoir (d. h. der WirkstoffMatrix) durch die Mikroprotrusionen in die Haut ermöglicht, so dass auch in diesem Fall - wie auch bei den Hohlnadeln - eine direkte Abgabe von Wirkstoffen in die Haut möglich ist.

Die Mikroprotrusionen können aus dem Fachmann bekannten, biokompatiblen und hautverträglichen Materialien, insbesondere Kunststoffen und Metallen, hergestellt werden.
Als Kunststoff-Materialien kommen beispielsweise in Betracht, einzeln oder in Kombination: Acrylonitril-StyrolCopolymere, Polymethylmethacrylate, PVC, Polytetrafluorethylen, Polyamide, Polyurethan, Polystyrol.
Als metallische Materialien kommen beispielsweise in Betracht: Edelstahl, Titan und Titan-Legierungen; Aluminium und -legierungen; Legierungen aus Kobalt, Chrom und Molybdän.
Die Mikroprotrusionen können auf dem Fachmann bekannte Weise durch Spritzguss, Formpressen, Thermoformung, Tiefziehen, Extrusion, Ätztechniken etc. erzeugt werden.

Die Erfindung schließt aber auch solche Ausführungsformen mit ein, bei denen die Wirkstoffabgabe nicht direkt über die Mikroprotrusionen erfolgt, sondern über die hautseitige Oberfläche des Wirkstoffreservoirs. In diesem Fall beschränkt sich die Funktion der Mikroprotrusionen auf das Durchbrechen der Hautbarriere und das Verankern der Vorrichtung in der Haut.

Gemäß einer weiteren Ausführungsform ist vorgesehen, dass die Vorrichtung auf der Hautseite eine klebende Polymermatrix aufweist, die vorzugsweise flächengleich mit der Ebene der Mikroprotrusionen angeordnet ist. Durch diese Maßnahme wird eine noch stärkere Befestigung der Vorrichtung auf der Haut ermöglicht. Die Mikroprotrusionen ragen dabei vorzugsweise durchschnittlich um weniger als 300 µm, insbesondere um weniger als 200 µm, aus der Ebene der Polymermatrixschicht heraus. Die klebende Polymermatrix kann zugleich das Wirkstoffreservoir bilden und einen oder mehrere Wirkstoffe, wahlweise in Kombination mit einem oder mehreren Hilfsstoffen, enthalten. Alternativ kann die klebende Polymermatrix wirkstofffrei sein, wobei der/die Wirkstoffe in einer oder mehreren zusätzlichen Schichten enthalten ist/sind.

Als Grundmaterial für die Herstellung der genannten Polymer-Matrix, des wirkstoffhaltigen Reservoirs oder einer haftklebenden Schicht eignen sich insbesondere folgende Polymere, einzeln oder in Kombination: Poly(meth)acrylate, Polyisobutylene, Polyterpene, Ethylenvinylacetat-Copolymere, Synthesekautschuke, Styrol-Isopren-Styrol-Blockcopolymere, Styrol-Butadien-Styrol-Blockcopolymere, Heißschmelzkleber, Mischungen aus Kautschuken und Harzen, Silikonhaftkleber, Polyvinylacetat, Polyvinylpyrrolidone, Polyvinylalkohole, Polyethylenglykole, Cellulosederivate (wie z. B. Hydroxypropylmethylcellulose). Haftklebende Formulierungen auf Basis der genannten Polymere sind dem Fachmann grundsätzlich bekannt.
Die Polymermatrix bzw. das Wirkstoffreservoir können ferner einen oder mehrere bekannte Hilfsstoffe enthalten, insbesondere aus der Gruppe der Weichmacher, Emulgatoren, Permeations-Enhancer, Klebrigmacher, Lösungsvermittler, Stabilisatoren, Füllstoffe und Trägerstoffe.

Die Polymermatrix weist vorzugsweise einen Polymer-Anteil von 10 bis 90 Gew.-%, insbesondere 30 bis 70 Gew.-% auf; der Anteil der Hilfsstoffe liegt bevorzugt zwischen 0,1 und 30 Gew.-%, insbesondere zwischen 1 und 10 Gew.-%. Der Wirkstoff-Gehalt liegt vorzugsweise im Bereich von 0,1 bis 20 Gew.-%., insbesondere von 0,5 bis 10 Gew.-%. Der Gewichtsanteil der Mikroprotrusionen wird bei der Berechnung nicht berücksichtigt.

Als Rückschicht oder Stützfolie eignen sich vor allem Polyester-Folien, welche sich durch besondere Festigkeit auszeichnen, wie z. B. Polyethylenterephthalat und Polybutylenterephthalat, darüber hinaus aber auch andere hautverträgliche Kunststoffmaterialien, wie z. B. Polyvinylchlorid, Ethylenvinylacetat, Vinylacetat, Polyethylen, Polypropylen und Cellulosederivate und viele andere mehr.

Die hautseitige Oberfläche der erfindungsgemäßen Vorrichtungen ist vorzugsweise mit einer ablösbaren Schutzfolie bedeckt, die vor der Applikation abgezogen wird. Für die ablösbare Schutzfolie können dieselben Materialien verwendet werden wie für die Rückschicht, vorausgesetzt, dass diese Schicht einer geeigneten Oberflächenbehandlung, z. B. Silikonisierung oder Fluorosilikonisierung, unterzogen wird. Es können aber auch andere ablösbare Schutzschichten, wie Polytetrafluorethylen-behandeltes Papier, Cellophan, Polyvinylchlorid, oder ähnliche verwendet werden.

Die erfindungsgemäßen Vorrichtungen eignen sich in vorteilhafter Weise zur transdermalen Verabreichung von pharmazeutischen Wirkstoffen oder Impfstoffen zur therapeutischen oder prophylaktischen Behandlung, auch zum Zwecke der Immunisierung, von Menschen oder Tieren. Sie eignen sich insbesondere zur Verabreichung von höhermolekularen oder stark polaren Wirkstoffen mit wirksamen oralen Dosierungen (Mensch) von weniger als 10 mg/Tag.
Die erfindungsgemäßen Vorrichtungen zeichnen sich einerseits durch eine sichere und langandauernde Haftung auf der Haut aus, andererseits ermöglichen sie die transdermale Verabreichung von solchen Wirkstoffen, die ansonsten für die transdermale Verabreichung nicht geeignet wären.

Vorzugsweise enthalten diese Vorrichtungen deshalb einen oder mehrere Wirkstoffe, der/die aus den Gruppen der Peptide (insbesondere Peptidhormone wie z. B. Insulin, Oxytocin, Vasopressin; Wachstumsfaktoren, Immunmodulatoren, Antibiotica), Proteine (z. B. Enzyme, Interferone, Interleukine, Antikörper), hochaktive gentechnologisch hergestellte Wirkstoffe; Oligonucleotide (z. B. Antisense-Oligonucleotide) und Polynucleotide (z. B. Plasmide) ausgewählt ist/ sind, oder/und sie enthalten einen oder mehrere Aktiv-Impfstoffe, vorzugsweise ausgewählt aus der Gruppe, die lebende Bakterien, abgetötete Bakterien, attenuierte oder gentechnisch veränderte Viren, inaktivierte Viren, bakterielle Toxoide, Proteine, Glykoproteine, gentechnisch hergestellte Antigene sowie Oligo- und Polynucleotide umfaßt.

Daneben können die erfindungsgemäßen Vorrichtungen auch zur Verabreichung anderer pharmazeutischer Wirkstoffe verwendet werden. Hierfür kommen insbesondere Wirkstoffe aus folgenden Gruppen in Betracht:

Blutdrucksenkende oder -regulierende Mittel; herzwirksame Mittel (insbesondere beta-Blocker und Anti-Arrhythmica); Analgetica; Steroidhormone; Anaesthetica; Appetitzügler; Anti-Allergica, Antihistaminica; anti-arteriosklerotische Wirkstoffe; anti-arthritische / antirheumatische Wirkstoffe; Antibiotica; Anticholinergica; Anticonvulsiva; Antidepressiva; antidiabetische Mittel; antidiarrhöische Wirkstoffe; Antidiuretica; Anti-Estrogene; Antimykotica / fungizide Wirkstoffe, Wirkstoffe gegen Gicht; Lipidsenker; Hormone; nicht-steroidale Antiphlogistica; Anti-Migränewirkstoffe; Wirkstoffe gegen Übelkeit; antineoplastische Wirkstoffe; Anti-Parkinson-Wirkstoffe, antipsychotische Wirkstoffe; antispastische / krampflösende Wirkstoffe; Antithrombotica; antivirale Wirkstoffe; Anxiolytica; Bronchodilatatoren; Calciumkanal-Blocker; Cholinergica; Cholinesterase-Inhibitoren; ZNS-Stimulantien; Dopaminrezeptor-Agonisten; Immunomodulatoren, Immunsupprimierende Wirkstoffe; Ionenaustauscher-Harze; Monoaminoxidase-Inhibitoren; Sedativa / Hypnotica; Thrombolytica; Vasodilatatoren; Vitamine.

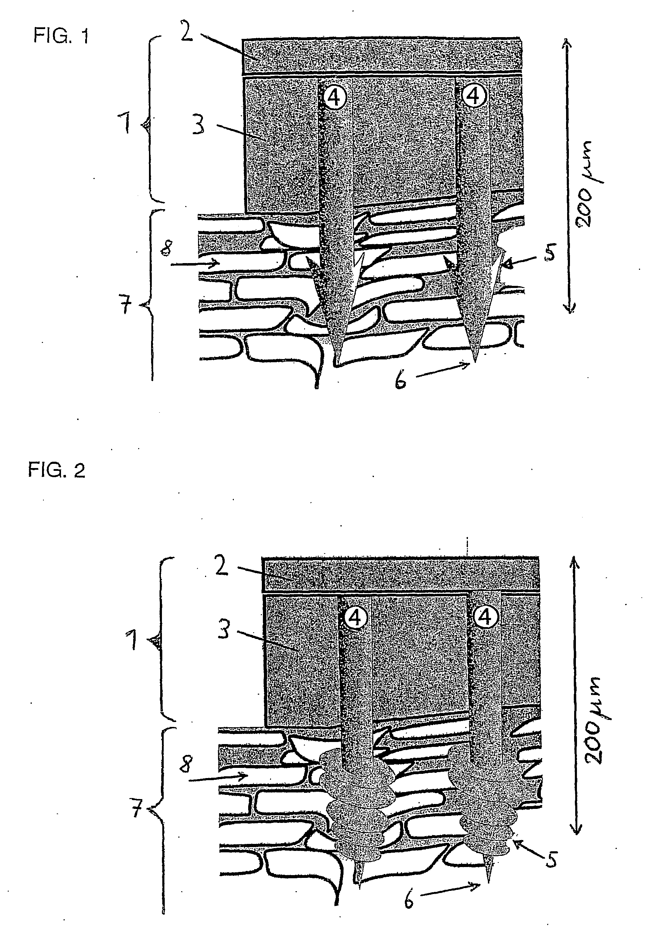

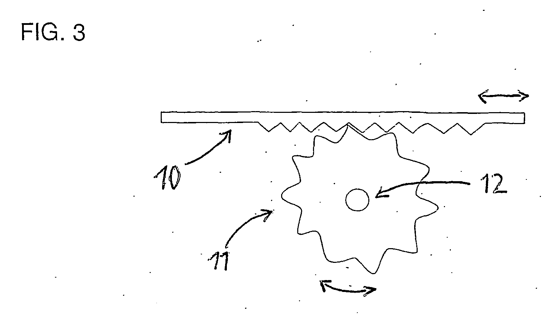

Die Erfindung wird anhand der schematischen Darstellungen der Fig. 1 bis 3 näher erläutert. Die dort gezeigten Ausführungsformen haben lediglich beispielhaften Charakter.

Fig. 1 und 2 zeigen jeweils eine Längsschnitt-Darstellung einer erfindungsgemäßen Vorrichtung (1) zur transdermalen Wirkstoff-Verabreichung, die sich im Zustand der Applikation auf der Haut (7) befindet. (8) bezeichnet die in die Lipidmatrix der Haut eingelagerten Corneocyten.

Fig. 1 zeigt eine Vorrichtung (1), die eine Rückschicht oder Stützfolie (2) und eine wirkstoffhaltige Polymermatrix (3) aufweist. In dieser Matrix befindet sich eine Vielzahl von Mikroprotrusionen (4), von denen zwei abgebildet sind.

Die Mikroprotrusionen (4) weisen an ihrem distalen Ende ihres im wesentlichen zylindrischen Schaftes eine Spitze (6) auf, mit der sie in die Haut (7) eindringen. Das gegenüberliegende Ende der Mikroprotrusionen (4) ist mit der Rückschicht (2) verbunden, so dass die Mikroprotrusionen (4) annähernd senkrecht zur Ebene der Rückschicht (2) angeordnet sind.
Die Mikroprotrusionen weisen widerhakenförmige Verankerungsstrukturen (5) auf. Die Mikroprotrusionen sind im Querschnitt rund, ebenso wie die Verankerungsstruktur (5).

Fig. 2 zeigt eine Abwandlung der in Fig. 1 gezeigten Vorrichtung, wobei die Verankerungsstrukturen (5) der Mikroprotrusionen (4) schraubenförmig ausgebildet sind. Die Mikroprotrusionen sind rotierend geführt und werden durch mikromechanische Antriebsmittel (nicht gezeigt) in Rotation versetzt. Im übrigen haben die Bezugszeichen dieselbe Bedeutung wie in Fig. 1.

Fig. 3 zeigt (in Schnitt-Darstellung) ein Beispiel eines mikromechanischen Aktors, der in einer erfindungsgemäßen Vorrichtung als Antriebsmittel verwendet werden kann, beispielsweise in einer Vorrichtung nach Fig. 2.
Durch Bewegung einer Mikro-Zahnstange (10) in einer der Pfeilrichtungen wird der Antrieb des Mikro-Zahnrades (11) bewirkt. Die Achse (12) des Zahnrades kann, wie in Fig. 2 gezeigt, in ihrem distalen (der Haut zugewandten) Bereich mit einer schraubenförmigen Verankerungsstruktur ausgestattet sein.
Durch Verwendung einer Vielzahl von Zahnstangen (10) und Zahnrädern (11) kann ein gleichsinniger Antrieb einer Vielzahl von schraubenförmigen Mikroprotrusionen (4, 5) bewirkt werden. Dabei besteht ferner die Möglichkeit, dass durch jeweils eine Zahnstange (10) zwei oder mehrere Zahnräder (11) gemeinsam angetrieben werden.

## Patentansprüche

1. Vorrichtung zur transdermalen Verabreichung von Wirkstoffen, mit einer Rückschicht und einem damit verbundenen wirkstoffhaltigen Reservoir, **dadurch gekennzeichnet, dass** die hautseitige Kontaktfläche der Vorrichtung eine Vielzahl von nadelförmigen Mikroprotrusionen aufweist, die zum Eindringen in die Haut geeignet sind, deren Längskontur eine oder mehrere Hinterschneidungen aufweist, welche das Herausziehen der Protrusionen aus der Haut erschweren und die Vorrichtung auf der Haut fixieren.

2. Vorrichtung nach Anspruch 1, **dadurch gekennzeichnet, dass** sie eine Vielzahl von Mikroprotrusionen aufweist, bei denen die genannten Strukturen als Widerhaken ausgebildet sind, wobei jede dieser Mikroprotrusionen einen oder mehrere solcher Haken aufweist.

3. Vorrichtung nach Anspruch 1, **dadurch gekennzeichnet, dass** sie eine Vielzahl von Mikroprotrusionen aufweist, die schraubenförmig gestaltet und drehbar angeordnet sind und **dadurch** beim Anwenden einer rotierenden Bewegung das Eindringen in die Haut erleichtern und eine Verankerung in der Haut bewirken.

4. Vorrichtung nach Anspruch 3, **dadurch gekennzeichnet, dass** der Drehantrieb durch mikromechanische Aktoren bewirkt wird.

5. Vorrichtung nach einem der vorangehenden Ansprüche, **dadurch gekennzeichnet, dass** die Mikroprotrusionen, oder zumindest einige davon, in dem wirkstoffhaltigen Reservoir befestigt sind.

6. Vorrichtung nach einem der vorangehenden Ansprüche, **dadurch gekennzeichnet, dass** die Mikroprotrusionen, oder zumindest einige der Mikroprotrusionen, mit der Rückschicht verbunden sind.

7. Vorrichtung nach einem der vorangehenden Ansprüche, **dadurch gekennzeichnet, dass** die Mikroprotrusionen, oder zumindest einige der Mikroprotrusionen, als Hohlnadeln ausgeführt sind.

8. Vorrichtung nach einem der vorangehenden Ansprüche, **dadurch gekennzeichnet, dass** sie auf der Hautseite eine klebende Polymermatrix aufweist, die vorzugsweise flächengleich mit der Ebene der Mikroprotrusionen angeordnet ist.

9. Vorrichtung nach Anspruch 8, **dadurch gekennzeichnet, dass** die Mikroprotrusionen durchschnittlich um weniger als 300 µm aus der Ebene der Polymermatrixschicht herausragen.

10. Vorrichtung nach Anspruch 8 oder 9, **dadurch gekennzeichnet, dass** die klebende Polymermatrix zugleich das Wirkstoffreservoir bildet und einen oder mehrere Wirkstoffe, wahlweise in Kombination mit einem oder mehreren Hilfsstoffen, enthält.

11. Vorrichtung nach einem der vorangehenden Ansprüche, **dadurch gekennzeichnet, dass** sie einen oder mehrere wirkstoffe enthält, der/die aus den Gruppen der Peptide, Proteine, oligonucleotide und Polynucleotide ausgewählt ist/ sind.

12. Vorrichtung nach einem der vorangehenden Ansprüche, **dadurch gekennzeichnet, dass** sie einen oder mehrere Impfstoffe enthält, vorzugsweise ausgewählt aus der Gruppe, die Bakterien, Viren, bakterielle Toxoide, Oligo- und Polynucleotide sowie gentechnisch hergestellte Antigene umfaßt.

## Claims

1. Device for transdermal administration of active substances, having a back layer and an active substance-containing reservoir connected thereto, **characterized in that** the skin-facing contact surface of the device has a plurality of needle-shaped microprotrusions which are suitable for penetrating into the skin and whose longitudinal contour has one or more undercuts that render extraction of the protrusions from the skin more difficult and fix the device on the skin.

2. Device according to claim 1, **characterised in that** it has a plurality of microprotrusions wherein said structures are configured as barbs, each of said microprotrusions having one or more of such barbs.

3. Device according to claims 1, **characterised in that** it has a plurality of microprotrusions which are helically configured and rotatably arranged and which thereby, upon application of a rotating movement, facilitate penetration into the skin and effect anchorage in the skin.

4. Device according to claim 3 **characterised in that** the rotary drive is effected by micromechanical actuators.

5. Device according to any one of the preceding claims, **characterized in that** the microprotrusions, or at least several of the microprotrusions, are fixed in the active substance-containing reservoir.

6. Device according to any one of the preceding claims, **characterised in that** the microprotrusions, or at least several of the microprotrusions, are connected with the back layer.

7. Device according to any one of the preceding claims, **characterized in that** the microprotrusions, or at least several of the microprotrusions, are configured as hollow needles.

8. Device according to any one of the preceding claims, **characterised in that** on the skin side it has an adhesive polymer matrix which is preferably arranged such that it is coextensive with the plane of the microprotrusions.

9. Device according to claim 8, **characterised in that** the microprotrusions protrude from the plane of the polymer matrix layer by, on average, less than 300 µm.

10. Device according to claim 8 or 9, **characterised in that** the adhesive polymer matrix at the same time constitutes the active substance reservoir and contains one or more active substances, optionally in combination with one or more auxiliary agents.

11. Device according to any one of the preceding claims, **characterised in that** it contains one or more active substances which is/are selected from the groups of the peptides, proteins, oligonucleotides and polynucleotides.

12. Device according to any one of the preceding claims, **characterised in that** it contains one or more vaccines preferably selected from the group comprising bacteria, viruses, bacterial toxoids, oligonucleotides and polynucleotides as well as genetically engineered antigens.

## Revendications

1. Dispositif pour l'administration transdermique de substances actives avec une rétrocouche et un réservoir contenant une ou plusieurs substances actives et relié à cette couche, **caractérisé en ce que** la surface de contact côté peau du dispositif présente une pluralité de microprotusions aciculaires, qui conviennent pour pénétrer dans la peau, dont le contour longitudinal présente une ou plusieurs contre-dépouilles, lesquelles rendent difficile l'extraction des protusions de la peau et fixent le dispositif sur la peau.

2. Dispositif selon la revendication 1, **caractérisé en ce qu'**il présente une pluralité de microprotusions, sur lesquelles lesdites structures sont conçues sous forme de crochets, chacune de ces microprotusions présentant un ou plusieurs crochets de ce type.

3. Dispositif selon la revendication 1, **caractérisé en ce qu'**il présente une pluralité de microprotusions, qui sont conçues en forme de vis et sont disposées de façon rotative et facilitent ainsi, lors de l'application d'un mouvement rotatif, la pénétration dans la peau et entraînent un ancrage dans la peau.

4. Dispositif selon la revendication 3, **caractérisé en ce que** l'entraînement de rotation est provoqué par des acteurs micromécaniques.

5. Dispositif selon l'une quelconque des revendications précédentes, **caractérisé en ce que** les microprotusions, au moins certaines d'entre elles, sont fixées dans le réservoir contenant une ou plusieurs substances actives.

6. Dispositif selon l'une quelconque des revendications précédentes, **caractérisé en ce que** les microprotusions ou au moins certaines des microprotusions sont reliées à la rétrocouche.

7. Dispositif selon l'une quelconque des revendications précédentes, **caractérisé en ce que** les microprotusions, tout du moins certaines des microprotusions sont réalisées sous forme d'aiguilles creuses.

8. Dispositif selon l'une quelconque des revendications précédentes, **caractérisé en ce qu'**il présente côté peau une matrice polymère adhésive, qui est disposée de préférence sur la même surface que le plan des microprotusions.

9. Dispositif selon la revendication 8, **caractérisé en ce que** les microprotusions dépassent en moyenne de moins de 300 µm du plan de la couche de matrice polymère.

10. Dispositif selon la revendication 8 ou 9, **caractérisé en ce que** la matrice polymère adhésive forme en même temps le réservoir de substance active et contient une ou plusieurs substances actives, en option en combinaison avec une ou plusieurs substances auxiliaires.

11. Dispositif selon l'une quelconque des revendications précédentes, **caractérisé en ce qu'**il contient une ou plusieurs substances actives, qui est/sont choisie(s) dans les groupes des peptides, protéines, oligonucléotides et polynucléotides.

12. Dispositif selon l'une quelconque des revendications précédentes, **caractérisé en ce qu'**il contient un ou plusieurs vaccins, de préférence choisis dans le groupe qui comprend des bactéries, des virus, des toxoïdes bactériens, des oligonucléotides et polynucléotides ainsi que des antigènes fabriqués par le génie génétique.
